# EUROPEAN PATENT APPLICATION

(11) **EP 4 681 519 A1**
(43) Date of publication of application: **21.01.2026**
(21) Application number: 25185479.0
(22) Date of filing: 26.06.2025
(51) Int. Cl.: A01C 21/00, A01M 7/00

(54) **A SENSOR SYSTEM FOR DYNAMIC AGRICULTURE NUTRIENT APPLICATION**

(30) Priority: 19.07.2024 US 202418777858
(71) Applicant: Deere & Company, Moline, IL 61265 (US)
(72) Inventor: Goering, Kevin J., Mannheim (DE); Wright, Yancy E., Mannheim (DE); Rees, Steven, Mannheim (DE)
(74) Representative: Holst, Sönke

(57) **Abstract**

A method that includes determining, based on data from a first sensor (204), a first estimated value of a crop health parameter in a first section (222) of a crop (220) and determining, based on data from a second sensor (206), a second estimated value of the crop health parameter in a second section (224) of the crop (220). The method also includes measuring, using a third sensor (208), a measured value of the crop health parameter in the second section, and comparing the second estimated value and the measured value to determine a calibration factor. Additionally, the method includes determining a first product application plan for the first section based upon the first estimated value and the calibration factor.

## Description

### TECHNICAL FIELD

This disclosure relates to a sensor system, and more particularly, to a sensor system for dynamic agriculture nutrient application.

### BACKGROUND

Selection, application, and timing of nutrients for growing crops is essential in agriculture production. Technology has improved the ability to deliver more precise levels (e.g., amounts) of nutrients to crops, thereby improving the overall yield when harvesting the crops. For example, positioning systems, such as a global positioning system (GPS), may be utilized by farmers to map a nutrient content of the soil within a field. The farmers may then utilize the nutrient map to deliver various amounts of nutrients, such as fertilizer, to particular portions of the field determined to be nutrient deficient based on the nutrient map. As a result, the farmers may no longer need to apply a set amount of the nutrients (e.g., the fertilizer) over the entire field.

### SUMMARY

In one aspect of the present disclosure, a method is disclosed. The method includes determining, based on data from a first sensor, a first estimated value of a crop health parameter in a first section of a crop and determining, based on data from a second sensor, a second estimated crop value of the health parameter in a second section of the crop. The method also includes measuring, using a third sensor, a measured value of the crop health parameter in the second section, and comparing the second estimated value and the measured value to determine a calibration factor. Additionally, the method includes determining a first product application plan for the first section based upon the first estimated value and the calibration factor.

In certain configurations, the method may further include applying a first product according to the first product application plan to the first section.

In certain configurations, the first estimated value may be a first estimated nutrient level in the first section, the second estimated value may be a second estimated nutrient level in the second section, and the measured value may be a measured nutrient level in the second section.

In certain configurations, the first sensor and the second sensor may be visible light cameras and the third sensor may be a multispectral camera.

In certain configurations, the first sensor and the second sensor may be visible light cameras and the third sensor may be a near-infrared camera.

In certain configurations, the first sensor and the second sensor are multispectral cameras and the third sensor may be a near-infrared camera that includes a calibrated light source.

In certain configurations, the first sensor and the second sensor may be near-infrared cameras and the third sensor may be a near infrared sensor that includes a calibrated light source.

In certain configurations, the method may further include determining a second product application plan for the second section based upon the second estimated value and the calibration factor, and applying a second product according to the second product application plan to the second section.

In certain configurations, the first sensor and the second sensor may form an array of sensors that extends across a working width of a machine, and a width of the first section combined with a width of the second section form the working width of the machine.

In certain configurations, the method may further include detecting, using a height sensor, a height of the crop within the first section. The first product application plan may be further based upon the row width of the crop. The method may also include detecting, using the first sensor, a row width of the crop within the first section. The first product application may be further based upon the row width of the crop.

In another aspect of the present disclosure, a system for agriculture crop health monitoring is disclosed. The system includes a non-transitory memory and a processor configured to execute instructions stored in the non-transitory memory. The processor is configured to execute instructions to determine, based on data from a first sensor, a first estimated value of a crop health parameter in a first section of a crop and determine, based on data from a second sensor, a second estimated value of the crop health parameter in a second section of the crop. The processor is also configured to execute instructions to measure, using a third sensor, a measured value of the crop health parameter in the second section and compare the second estimated value and the measured value to determine a calibration factor. The processor is also configured to execute instructions to determine a first product application plan for the first section based upon the first estimated value and the calibration factor.

In certain configurations, the processor may be further configured to execute instructions to detect, using a height sensor, a height of the crop within the first section and detect, using the first sensor, a row width of the crop within the first section. Determining the first product application plan may be further based upon the height of the crop and the row width of the crop.

In certain configurations, the calibration factor may correspond to a difference between the second estimated value and the measured value.

In certain configurations, the process may be further configured to execute instructions to determine a second product application plan for the second section based upon the second estimated value and the calibration factor and apply a second product according to the second product application plan to the second section.

In certain configurations, the processor may be further configured to execute instructions to determine a second product application plan for the second section based upon the measured value and apply a second product according to the second product application plan to the second section.

In certain configurations, the processor may be further configured to execute instructions to transmit, via a network, information associated with the crop to a controller of a vehicle. The information associated with the crop may include at least one of the first estimated value, the second estimated value, the value, the calibration factor, and the first product application plan.

In certain configurations, the first sensor and the second sensor may be the same type of sensor. The third sensor may be a different type of sensor than the first sensor and the second sensor.

In another aspect of the present disclosure, a system for dynamic agriculture nutrient application is disclosed. The system includes an applicator configured to apply nutrients to a crop and a sensor system in communication with the applicator and configured to monitor the crop. The sensor system includes a first sensor configured to determine a first estimated nutrient level in a first section of the crop, a second sensor configured to determine a second estimated nutrient level in a second section of the crop, and a third sensor configured to measure a measured nutrient level in the second section. The sensor system is configured to compare the second estimated nutrient level and the measured nutrient level to determine a calibration factor, determine a first nutrient application plan for the first section based upon the first estimated nutrient level and the calibration factor, and communicate with the applicator such that the applicator applies first nutrients according to the first nutrient application plan to the first section.

In certain configurations, the calibration factor may correspond to a difference between the second estimated nutrient level and the measured nutrient level. Additionally, the sensor system may be configured to monitor the crop to adjust the first nutrient application plan in real-time.

### BRIEF DESCRIPTION OF THE DRAWINGS

The disclosure is best understood from the following detailed description when read in conjunction with the accompanying drawings. It is emphasized that, according to common practice, the various features of the drawings are not to scale. On the contrary, the dimensions of the various features are arbitrarily expanded or reduced for clarity.
FIG. 1A is a side view of an example of a machine that includes a sensor system.
FIG. 1B is a top-down view of the machine of FIG. 1A.
FIG. 2 is a schematic view of another example of a sensor system of a machine.
FIG. 3 is a diagram of an example of a sensor system of a machine.
FIG. 4 is a flowchart of an example of a process for agriculture product application using a sensor system.
FIG. 5 is a flowchart of an example of a process for agriculture nutrient application using a sensor system.
FIG. 6 is a flowchart of another example of a process for agriculture nutrient application using a sensor system.
FIG. 7 is a flowchart of an example of a process for agriculture product application using a sensor system.

### DETAILED DESCRIPTION

The present disclosure relates to a sensor system for use with various machinery, providing a cost-effective and accurate way of monitoring crops. The sensor system may be configured for use with agricultural machinery, construction equipment, automotive vehicles, or other types of machinery. By way of example, the sensor system may be configured for use with agricultural machinery, such as a planter machine configured to plant various seeds or a sprayer machine configured to apply one or more products (e.g., nutrients, fertilizers, pesticides, soil amendments, water, or growth regulators) to various crops.

The sensor system may be configured to detect and/or analyze a region surrounding the machinery (e.g., agricultural machinery) being operated. The sensor system may detect objects, movement, environmental conditions, crop conditions (e.g., crop health, crop nutrient levels, etc.), crop dimensions (e.g., crop width, crop height, crop row width, etc.), or a combination thereof, which may be analyzed to determine the performance of the associated machinery and/or to determine an overall condition of the region surrounding the machinery. For example, the sensor system may include one or more imaging sensors that may be configured to capture images and/or videos within a field of view of the sensors (e.g., in the region surrounding the machinery); one or more thermal cameras that may be configured to detect or measure heat patterns; one or more environmental sensors that may be configured to detect or measure environmental conditions (e.g., temperature, humidity, wind, soil moisture, etc.); one or more operational sensors that may be configured to detect or measure operating conditions of the machinery (e.g., movement of the machinery, machinery degradation, fuel levels, speed, GPS location, etc.); one or more other sensors, such as proximity sensors configured to detect obstacles or other machine nearby and/or acoustic sensors configured to identify machinery anomalies through sound analysis; other types of sensors; or a combination thereof.

Conventional crop monitoring may be performed or utilized to determine an overall status of various crops. For example, a nutrient level of the crops, a moisture level of the crops, a health level of the crops (e.g., fungal and/or insect presence), other crop-related parameters, or a combination thereof may be determined based on the crop monitoring. Such monitoring may typically be performed by collecting and evaluating soil samples (i.e., soil sampling). However, while soil sampling may provide information one or more of the above parameters prior to planting, at least some of these parameters often change during the growing season due to, for example, plant usage, growth, leaching, product applications (e.g., fertilizers, fungicides, pesticides, etc.), other factors, or a combination thereof. As such, soil sampling may be unable to provide an accurate measurement of one or more of the above parameters, which may result in overapplication and/or underapplication of various products (e.g., fertilizers, fungicides, or pesticides).

To more accurately measure crop-related parameters, such as those described above, conventional sensor systems may be implemented to monitor the crops throughout the growing season. For example, one or more sensors may be mounted on the machinery (e.g., the agricultural machinery), on mobile equipment such as a drone, or both. Additionally, satellite imaging may be used to monitor the crops throughout the growing season. However, none of these conventional solutions monitor crops in an accurate and repeatable manner. For example, because sensors only detect crop-related parameters within a limited field of view and/or due to variance between particular crops within a field, conventional sensor systems may be unable to consistently and accurately measure the crop-related parameters throughout the entire crop field.

Similarly, environmental conditions and/or positioning of the sensors on the machinery or the mobile equipment may also contribute to variability (e.g., accuracy) of the sensors. For example, the time of day, weather conditions (e.g., cloud coverage, fog, haze), or seasonal changes may affect an angle and/or an amount of sunlight (e.g., full sun, cloudy, shadows being casted by the sun) that reaches the sensors and/or reaches the crops. As a result, visibility of the crops by the sensors may be negatively impacted. Moreover, a direction of travel of the machinery or mobile equipment may also impact the accuracy of the sensors. For example, the sensors may have poor visibility when a direction of travel of the machinery or mobile equipment is directed towards or away from the sun.

To improve on the above challenges, other sampling techniques may be implemented. For example, plant tissue sampling may provide more accurate measurements of crop-related parameters. However, such sampling techniques may be labor intensive, time prohibitive, cost prohibitive, or a combination thereof.

The present teachings provide a sensor system that addresses the aforementioned challenges. The sensor system described herein may be configured to monitor crop-related parameters. The crop-related parameters may include one or more of nutrient levels of crops, a moisture level of the crops, a health level of the crops (e.g., fungal and/or insect presence), other crop-related parameters, or a combination thereof in real-time. The sensor system may also provide real-time feedback to the machinery associated with the sensor system. By way of example, the sensor system may monitor the crops during product (e.g., fertilizer, nutrients, fungicide, pesticide, etc.) application to the crops by an applicator, and the sensor system may provide feedback to the applicator (e.g., to one or more computing devices therein or associated therewith), which can be configured to adjust the application process accordingly. For example, the sensor system may determine a calibration factor, and the calibration factor may be provided to the applicator to adjust a rate of application of the product.

Moreover, the sensor system described herein may use multiple sensors to accurately sense one or more of crop-related parameters in real-time, enabling precise application of products (e.g., fertilizer, nutrients, fungicide, pesticide, etc.) to maximize yield and/or protein content while minimizing excess nutrient application. As a result, the sensor system may reduce overall costs associated with crop cultivation and minimize the environmental impact caused by overapplication of products (e.g., fertilizer, nutrients, fungicide, or pesticide).

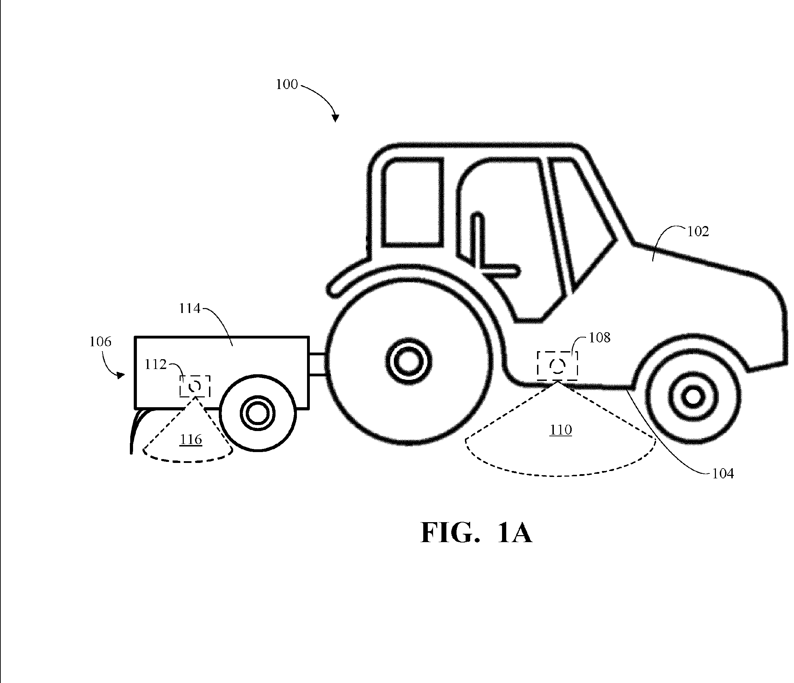

Turning now to the figures, FIG. 1A illustrates a side view of a machine 100 in accordance with the present teachings. The machine 100 may include a body 102 and a frame 104. The frame 104 may form part of the body 102 or may support the body 102 of the machine 100. Additionally, the machine 100 may include a sensor system 106. As discussed above, the machine 100 is not limited to any specific type of machinery. For example, the machine 100 may be a vehicle, equipment, other object, or a combination thereof. The machine 100 may be configured for operation in any type of industry including agriculture, horticulture, , or other types of industry. By way of example, as shown in FIG. 1A, the machine 100 may be an agriculture machine, such as a tractor or applicator that is configured to apply one or more products (e.g., fertilizer, nutrients, fungicide, pesticide, etc.) to crops.

The sensor system 106 may be coupled to the machine 100 so that one or more sensors of the sensor system 106, such as a first sensor 108, may be positioned to monitor or otherwise interact with an area around the machine 100. For example, the first sensor 108 may be coupled to the body 102 or the frame 104 of the machine 100, such as along an outer region or outer surface of the frame 104. Thus, a field of view 110 of the first sensor 108 (e.g., a field of view of a lens of the first sensor 108) may extend outboard with respect to the machine 100 along the ground beneath the machine 100.

Alternatively, or additionally, the sensor system 106 may be coupled to an accessory or secondary component of the machine 100. For example, as shown in FIG. 1A, the sensor system 106 may include a second sensor 112 coupled to a trailer 114 of the machine 100. The trailer 114 may be connected to the machine 100 so that the trailer 114 may, for example, be towed or pushed by the machine 100. For example, the trailer 114 may be an applicator that is configured to apply water, nutrients, fertilizer, pesticides, fungicides, or a combination thereof to the crops. As such, the second sensor 112 may be positioned external to the machine 100 so that a field of view 116 of the second sensor 112 (e.g., a field of view of a lens of the second sensor 112) may extend outboard with respect to the trailer 114 along the ground beneath the trailer 114.

It should be noted that the sensor system 106 may include any number of sensors (e.g., zero or more of the first sensor 108, zero or more of the second sensor 112, one or more additional sensors, etc.) and may be positioned in any desired manner by adjusting the mounting of the sensor system 106 to the machine 100 and/or to the trailer 114. That is, an overall field of view, which may include the field of view 110 of the first sensor 108 and/or the field of view 116 of the second sensor 112, may be configured to capture any desired region around the machine 100, which may include parts or components of the machine 100 and/or the trailer 114, surrounding crops, the ground surround the machine 100 and/or the trailer 114, or a combination thereof.

By way of example, the machine 100 may be coupled to the trailer 114 to drive the trailer 114, where the trailer 114 may be an applicator configured to apply a topical spray to existing crops within a field, such as a pesticide or fertilizer. In this configuration, the sensor system 106 may be coupled to the machine 100 and/or the trailer 114 so that the field of view 110 of the first sensor 108 and/or the field of view 116 of the second sensor 112 can monitor the crops within the field. The first sensor 108 and the second sensor 112 may be positioned to monitor various crop-related parameters (e.g., sensor data associated with the crops) and/or monitor operation of the machine 100 (e.g., sensor data associated with the operation of the machine 100). The first sensor 108 and the second sensor 112 may capture the sensor data associated with the crops, which may then be evaluated by the sensor system 106 (e.g., a computing device within the sensor system 106) or by a device in communication with the sensor system 106 (e.g., a computing device or system of the machine 100 in communication with the sensor system 106) to determine a current condition (e.g., health) of the crops. Captured sensor data associated with the operation of the machine 100 may be evaluated by the sensor system 106 (e.g., a computing device within the sensor system 106) or a device in communication with the sensor system 106 (e.g., a computing device or system of the machine 100 in communication with the sensor system 106) to determine the quality of performance of the machine 100.

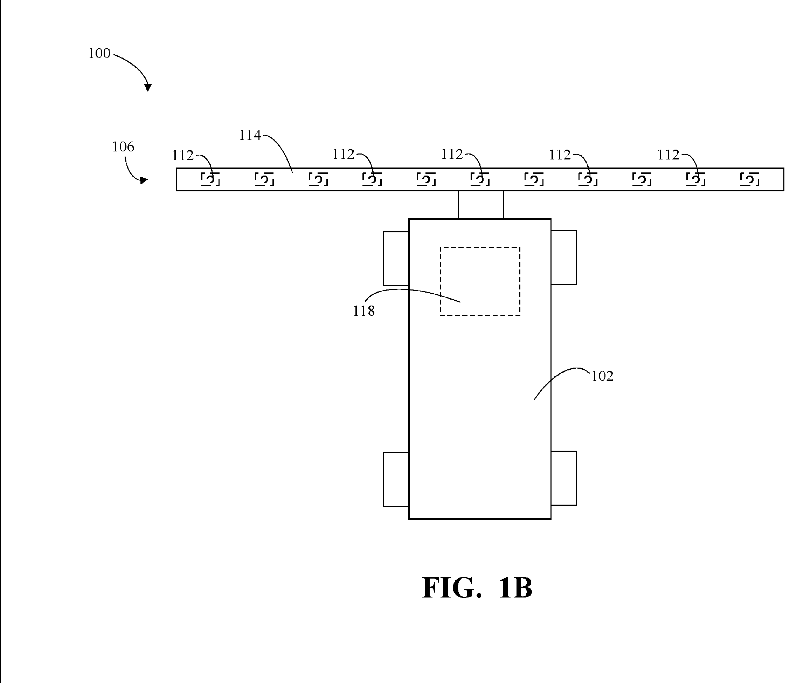

FIG. 1B illustrates a top-down view of the machine 100 and the trailer 114 shown in FIG. 1A. As discussed above, the sensor system 106 may include more than one of the first sensor 108 and/or more than one of the second sensor 112. In certain configurations, the sensor system 106 may not include the first sensor 108 or may not include the second sensor 112.

By way of example, FIG. 1B illustrates an array of the second sensor 112. The array includes a plurality of the second sensors 112, which may extend along substantially the entire width of the trailer 114, as measured transverse to a direction of travel of the machine 100 and the trailer 114 during operation. Each of the sensors of the array of the second sensors 112 may include a field of view that may be similar to the field of view 116 shown in FIG. 1A. As a result, the array of the second sensors 112 may monitor and capture data (e.g., data associated with the crops and/or data associated with the operation of the machine 100) in a surrounding area that extends along the width of the trailer 114. The captured data may then be evaluated by the sensor system 106 (e.g., a computing device within the sensor system 106) or a device in communication with the sensor system 106 (e.g., a computing device or system of the machine 100 in communication with the sensor system 106). For example, the captured data may be initially transmitted from the second sensors 112 to a controller (not shown) of the sensor system 106, which may in turn transmit the captured data to a controller 118 of the machine 100. In such a case, the controller of the sensor system 106 and/or the controller 118 of the machine 100 may evaluate the captured data.

It should be noted that, while the trailer 114 is described in detail above, implementation of the sensor system 106 is not limited to the trailer 114. By way of example, the sensor system 106 may be implemented on self-propelled machinery, such as a self-propelled sprayer or applicator. For example, the sensor system 106 may include one or more sensors that may be positioned along a boom of the self-propelled machinery such that the one or more sensors may be positioned on at least one of a front portion, a rear portion, one or more side portions, a top portion, and a bottom portion of the machinery (e.g., on the boom of the machinery). As such, the sensor system 106 may be configured to monitor a direction of travel of the machinery or other directions transverse to the direction of travel of the machinery.

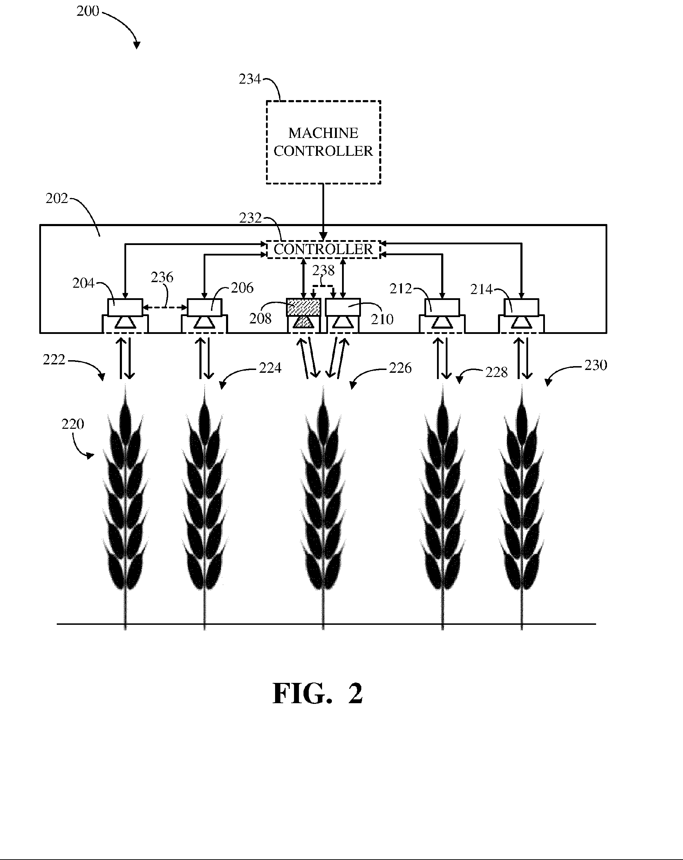

FIG. 2 illustrates a schematic view of another example of a sensor system 200 in accordance with the present disclosure. The sensor system 200 may be similar to the sensor system 106 of the machine 100 described above and shown in FIGS. 1A and 1B.

The sensor system 200 may be coupled to or otherwise disposed along an apparatus 202 of a machine. For example, the apparatus 202 may be similar to the trailer 114 of the machine 100 described above and shown in FIGS. 1A and 1B. The apparatus 202 may be a planting apparatus that is configured to be towed a machine (e.g., the machine 100) to plant seeds within a field. The apparatus 202 may also be an applicator that may be coupled to a machine (e.g., the machine 100) and configured to apply one or more products (e.g., fertilizer, nutrients, fungicide, or pesticide) to crops within a crop field. It should be noted that the apparatus 202 is not particularly limited to any one configuration and may be any component or secondary device in communication with and/or coupled to a machine (e.g., the machine 100).

As shown in FIG. 2, the sensor system 200 may include a plurality of sensors disposed along the apparatus 202. The sensor system 200 may include a first sensor 204, a second sensor 206, a third sensor 208, a fourth sensor 210, a fifth sensor 212, and a sixth sensor 214. However, the sensor system 200 may include more or fewer sensors than the sensors 204-214 shown in FIG. 2. Each of the sensors 204-214 may be in communication with at least some of the other sensors (as indicated by the dashed line 236 extending between the first sensor 204 and the second sensor 206 and the dashed line 238 extending between the third sensor 208 and the fourth sensor 210). Each of the sensors 204-214 may be in communication with a controller 216 of the sensor system 200.

The sensors 204-214 may monitor (i.e., may be configured to monitor) a crop 220. The sensors 204-214 may monitor one or more sections of the crop 220. A section of the crop 220 may be a defined area or portion (e.g., a row or group of rows) of the crop 220. By way of example, the first sensor 204 may monitor a first section 222 of the crop 220, the second sensor 206 may monitor a second section 224 of the crop 220, the third sensor 208 and the fourth sensor 210 may monitor a third section 226 of the crop 220, the fifth sensor 212 may monitor a fourth section 228 of the crop 220, and the sixth sensor 214 may monitor a fifth section 230 of the crop 220. A single one of the sensors 204-214 may monitor a respective one of the sections 222-230 or more than one of the sensors 204-214 may monitor a single one of the sections 222-230. For example, as shown in FIG. 2, the third section 226 of the crop 220 may be monitored by both the third sensor 208 and the fourth sensor 210. In the interest of clarity, as described herein, the crop 220 may refer to a crop field in its entirety and may include a plurality of plants located within any one of the sections 222-230 described above.

The sensors 204-214 may be the same type of sensor or may be different types of sensors. The sensors 204-214 may be configured such that the sensors 204-214 are contained within or are in communication with one or more image capture devices of the sensor system 200 to capture still images and/or videos of the crop 220 within a respective one of the sections 222-230 of the crop 220. Each of the sensors 204-214 may be or may include an image capture device, whereby the sensors 204-214 are not limited to any one type of imaging sensor. For example, a sensor of the sensors 204-214 may be or may be part of a multispectral camera, a visible light camera, a near-infrared camera, an ultraviolet (UV) camera, a thermal camera, or some other type of camera.

The sensor system 200 may utilize the sensors 204-214 to monitor the crop 220 within each of the sections 222-230. In particular, the sensor system 200 may include different types of sensors and data captured by the sensors 204-214 of the sensor system 200 can be used to evaluate the overall accuracy of the sensor system 200 and/or the accuracy of each of the sensors 204-214 individually. That is, one or more of the sensors 204-214 may be utilized to validate and/or calibrate the sensor system 200 by validating and/or calibrating at least one of the sensors 204-214 based upon the data captured by at least some of the sensors 204-214.

By way of example, the first sensor 204, the second sensor 206, the fourth sensor 210, the fifth sensor 212, and the sixth sensor 214 may be the same type of sensor. For example, each of these sensors may be or may be part of a visible light camera, a multispectral camera, or a near-infrared camera. As such, these sensors may be configured to detect and capture data associated with the crop 220 in a similar manner within their respective sections 222-230.

The third sensor 208 may be a different type of sensor than the first sensor 204, the second sensor 206, the fourth sensor 210, the fifth sensor 212, and the sixth sensor 214 (as indicated by the hatching of the third sensor 208 shown in FIG. 2). For example, whereas the third sensor 208 may be or may be part of a visible light camera, a multispectral camera, or a near-infrared camera, its type must differ from that of the other sensors. In certain configurations the third sensor 208 may be the same type as the other sensors but may be configured differently. For example, all of the sensors 204-214 may be or may be part of a near-infrared sensor, whereby the third sensor 208 may include a calibrated light source such that the third sensor 208 may more accurately capture data when compared to the first sensor 204, the second sensor 206, the fourth sensor 210, the fifth sensor 212, and the sixth sensor 214.

Due to the third sensor 208 being a different type of sensor or configured differently than the other sensors, the third sensor 208 may be utilized to calibrate and/or validate the data captured by at least one of the other sensors (e.g., the first sensor 204, the second sensor 206, the fourth sensor 210, the fifth sensor 212, or the sixth sensor 214). For example, if the third sensor 208 is configured to more accurately capture data associated with the crop 220 in the third section 226 compared with the data associated to the crop 220 that is captured by the fourth sensor 210, the data captured by the third sensor 208 may be compared to the data captured by the fourth sensor 210. It should also be noted that the sensors may be partitioned into subsets of the sensor system 200. For example, the first sensor 204 and the second sensor 206 may be a first subset and the sensors 208-214 may be a second subset. In this configuration, the first subset and the second subset may each include one sensor that may be of a different type and/or a different configuration compared to the other sensors within their respective subset.

The data captured by both the third sensor 208 and the fourth sensor 210 may be associated with the same crop-related parameter such that the data captured by the third sensor 208 may be compared to the data captured by the fourth sensor 210 to determine a difference therebetween. For example, the data captured by the third sensor 208 may be associated with a nutrient level of the crop 220 in the third section 226 of the crop 220 and the data captured by the fourth sensor 210 may also be associated with the nutrient level of the crop 220 in the third section 226 of the crop 220. The data captured by the third sensor 208 may be compared to the data captured by the fourth sensor 210 to determine the accuracy of the fourth sensor 210.

By way of example, the crop 220 may reflect light (e.g., light from the sun or from an artificial light source) in various distinct patterns based upon a physiological state of the crop 220 (e.g., health of the crop 220 or stress of the crop 220 due to a lack of nutrients or other crop-related parameters). That is, the pattern of light reflected by the crop 220 may vary based upon a current physiological condition of the crop 220 and may provide specific signatures (e.g., spectral signatures) associated with different physiological conditions (e.g., nutrient levels) of the crop 220.

To capture the aforementioned spectral signatures, the third sensor 208 may be or may be part of a multispectral camera that is configured to capture data in the form of one or more images in multiple spectral bands at wavelengths across a range of the electromagnetic spectrum, including visible light (e.g., wavelengths of about 380 nm to about 750 nm) and near-infrared (e.g., wavelengths of about 750 nm to about 1400 nm). The third sensor 208 may capture the light that is reflected off of the third section 226 of the crop 220 in the form of one or more images, and the captured image(s) may be evaluated by the sensor system 200 or a system in communication with the sensor system 200 to determine the physiological state of the crop 220 within the third section 226.

For example, the captured image(s) by the third sensor 208 may be evaluated to identify the specific spectral signatures within the third section 226 of the crop 220. Evaluation of the captured image(s) may include, for example, analyzing the image(s) to identify any distinct reflectance patterns, determining one or more reflectance values based upon the captured image(s), and calculating one or more crop-related parameters based upon the determined reflectance value(s).

Additionally, the fourth sensor 210 may be or may be part of a visible light camera that is configured to capture data in the form of one or more images only in the visible light spectral band (e.g., wavelengths of about 380 nm to about 750 nm). The fourth sensor 210 may capture the light that is reflected off of the third section 226 of the crop 220 in the form of one or more images, and the captured image(s) may be evaluated by the sensor system 200 or a system in communication with the sensor system 200 to determine the physiological state of the crop 220 (e.g., the overall physiological state of the crop 220 based upon the crop-related parameters) within the third section 226. Evaluation of the image(s) captured by the fourth sensor 210 may be similar to the evaluation of the image(s) captured by the third sensor 208. However, due to the fourth sensor 210 being unable to capture images in spectral bands other than the visible light spectral band (e.g., unable to capture images in the near-infrared spectral band), evaluation of the images captured by the fourth sensor 210 may be unable to identify certain physiological conditions of the crop 220 within the third section 226. For example, if certain spectral signatures are only present in the near-infrared spectral band, such spectral signatures would not be identified by evaluating the image(s) captured by the fourth sensor 210 and may only be identified by evaluating the image(s) captured by the third sensor 208.

Due to the above potential discrepancies between the evaluation of the image(s) captured by the third sensor 208 and the image(s) captured by the fourth sensor 210, the third sensor 208 may be considered more accurate when compared to the fourth sensor 210 and its captured image(s). As a result, the image(s) captured by the third sensor 208 and/or data extracted therefrom (e.g., distinct reflectance patterns, one or more reflectance values, or one or more crop-related parameters) may be compared to the image(s) captured by the fourth sensor 210 and/or data extracted therefrom (e.g., distinct reflective patterns, one or more reflectance values, or one or more crop-related parameters) to determine an accuracy of the fourth sensor 210.

As discussed above, the fourth sensor 210 may be the same type or configuration of sensor as the first sensor 204, the second sensor 206, the fifth sensor 212, and the sixth sensor 214. As such, the accuracy determined for the fourth sensor 210 based upon the above evaluation may be used to determine a calibration factor to formulate tailored product (e.g., fertilizer, fungicide, or pesticide) application plans for different crop sections.

For example, a controller 232 of the sensor system 200 may compare the image(s) captured by the third sensor 208 and/or data extracted therefrom to the image(s) captured by the fourth sensor 210 and/or data extracted therefrom to determine the calibration factors to adjust the an application plans for different crop sections The controller 232 may also communicate with other components of the apparatus 202 to modify one or more operating parameters of the apparatus 202 in each of the sections 222-230 based upon the calibration factor. For example, the apparatus 202 may be an applicator and the controller 232 may communicate to a portion of the applicator (e.g., a sprayer) located in each of the sections 222-230 to modify an application rate in each of the sections 222-230 based upon the calibration factor. Thus, the controller 232 may selectively and differently modify application parameters of a product within at least some of the sections 222-230 based on the data captured by the sensor system 200 and based upon a comparison of the data as described above.

The controller 232 of the apparatus 202 may also be in communication with a machine controller 234 of the machine (e.g., the machine 100). The machine controller 234 of the machine may be similar to the controller 118 of the machine 100 shown in FIGS. 1A and 1B. The machine controller 234 of the machine may receive the data captured by the sensors 204-214 for evaluation. The machine controller 234 may then provide such data to a user of the machine (e.g., the machine 100), such as an operator, via a display or other means of communication. Additionally, the machine controller 234 may communicate with the controller 232 of the sensor system 200 to operate the sensors 204-214, modify one or more operating parameters of the sensors 204-214, modify one or more operating parameters of the apparatus 202, or a combination thereof. Thus, the machine (e.g., the machine 100) may maintain communication with the apparatus 202 based upon communication between the machine controller 234 of the machine and the controller 232 of the sensor system 200.

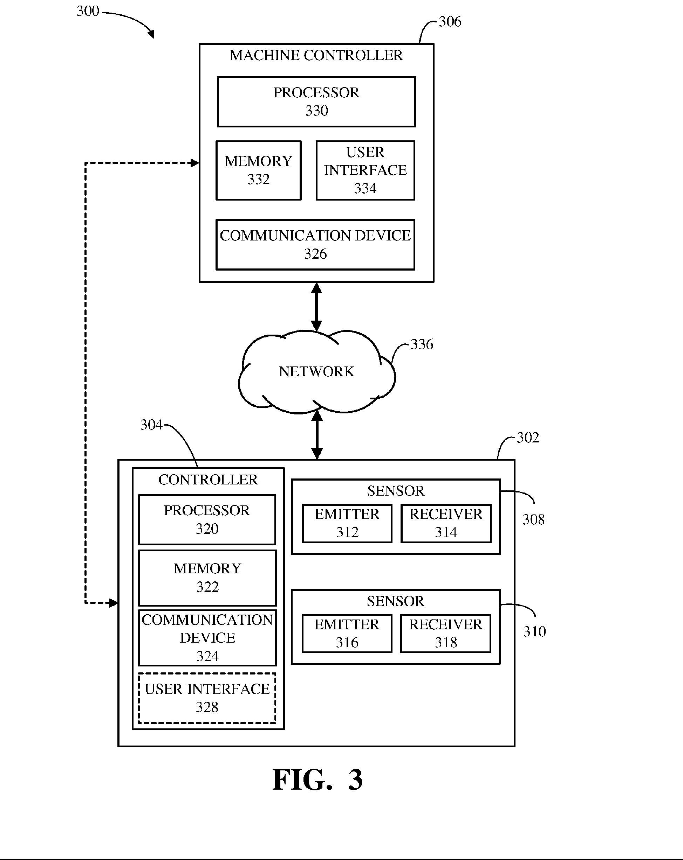

To describe some implementations of the sensor systems described herein, FIG. 3 is a diagram of an example of a system 300 to further illustrate the electronic computing and communication of the sensor systems described herein. For example, the system 300 may include a sensor system 302, which may be or may be similar to the sensor system 106 shown in FIGS. 1A and 1B or the sensor system 200 shown in FIG. 2.

The sensor system 30 may include one or more computing devices, such as a controller 304 of the sensor system 302, a machine controller 306 in communication with the controller 304 of the sensor system 302, or both.

The system 300 may include one or more sensors, such as a first sensor 308 and a second sensor 310. The first sensor 308 and the second sensor 310 may be similar to the first sensor 108 and/or the second sensor 112 of the sensor system 106 shown in FIGS. 1A and 1B or may be similar to the sensors 204-214 of the sensor system 200 shown in FIG. 2. By way of example, the first sensor 308 may include an emitter 312 and a receiver 314. The first sensor 308 may be or may be part of an image sensor module (e.g., a camera) such that the emitter 312 may transmit light to a portion of a crop (e.g., the crop 220) within a field of view of the first sensor 308. The light may reflect off the crop within the field of view of the first sensor 308 and be captured by the receiver 314 in the form of one or more images. The captured image(s) may then be further evaluated to determine a physiological state and/or condition of the crop 220 with the field of view of the first sensor 308, such as by identifying distinct reflectance patterns, determining one or more reflectance values based upon the captured image(s), and calculating one or more crop-related parameters based upon the determined reflectance value(s). Such evaluation may be completed by the controller 304 of the sensor system 302.

The second sensor 310 may be or may be part of an image sensor module (e.g., a camera) that may be similar or different from the first sensor 308. The second sensor 310 may include an emitter 316 and a receiver 318. The emitter 316 may transmit light to a portion of a crop (e.g., the crop 220) within a field of view of the second sensor 310. The light may reflect off the crop within the field of view of the second sensor 310 and be captured by the receiver 318 in the form of one or more images. The captured image(s) may then be evaluated similar to the captured image(s) of the first sensor 308 to determine a physiological state and/or condition of the crop 220 with the field of view of the first sensor 308. Such evaluation may be completed by the controller 304 of the sensor system 302.

The controller 304 may be or may include a processor 320, such as a microprocessor, and may include a single processor or multiple processors. A processor may have a single processing core or multiple processing cores. Alternatively, the processor 320 may include other types of devices, or multiple devices, not existing or hereafter developed, configured for manipulating or processing information. The controller 304 may include multiple processors interconnected in one or more manners, including but not limited to, hardwired or networked (e.g., wirelessly networked). By way of example, the operations of the processor 320 may be distributed across multiple devices or units that may be coupled directly or via a local area or other suitable network. The processor 320 may also include a cache or cache memory for local storage of operating data or instructions associated with the evaluation (e.g., further processing) of the data captured by the first sensor 308 and/or the second sensor 310.

The controller 304 may also include a memory 322. The memory 322 may include one or more memory components, which may each be volatile memory or non-volatile memory. For example, the volatile memory of the memory 322 may be random access memory (RAM)(e.g., a DRAM module, such as DDR SDRAM) or another form of volatile memory. In another example, the non-volatile memory of the memory 322 may be a disk drive, a solid-state drive, flash memory, phase-change memory, or another form of non-volatile memory configured for persistent electronic information storage. The memory 322 may also include other types of devices, now existing or hereafter developed, configured for storing data or instructions for processing by the processor of the controller 304.

The memory 322 can include data for immediate access by the controller 304. For example, the memory 322 may include executable instructions, application data, an operating system, or a combination thereof accessible by the processor 320 of the controller 304. The executable instructions, the application data, the operating system, or a combination thereof may be loaded or copied, in whole or in part, from non-volatile memory to volatile memory to be executed by the controller 304. For example, the executable instructions and application data may include instructions and data for evaluating the data captured by the first sensor 308 and/or the second sensor 310.

The memory 322 may include executable instructions or application data associated with a communication device 324 of the controller 304 of the sensor system 302. The communication device 324 may be or may include a transmitter and/or a receiver. The communication device 324 may facilitate communication between the controller 304 of the sensor system 302 and the controller 304 of the machine. For example, the data (e.g., the images) captured by the first sensor 308 and/or the second sensor 310 may be evaluated by the controller 304 of the sensor system 302, and results of such evaluation may be transmitted to the machine controller 306 for review by an operator of the machine. The communication device 324 may communication with a communication device 326 of the machine controller 306, which may be similar to or different than the communication device 324.

In certain configurations, the controller 304 may include a user interface 328. The user interface 328 may include one or more input interfaces and/or output interfaces. An input interface may be, for example, a positional input device, such as a mouse, touchpad, touchscreen, or the like; a keyboard; or another suitable human or machine interface device. An output interface may be, for example, a display, such as a liquid crystal display, a cathode-ray tube, a light emitting diode display, or other suitable display. Thus, the operator may interact with the sensor system 302 to monitor and/or operate the sensor system 302.

As discussed above, the sensor system 302 may be in communication with the machine controller 306 of a machine that is coupled to, or that is in communication with, the sensor system 302. The machine may be similar to the machine 100 shown in FIGS. 1A and 1B. The machine controller 306 may receive and/or evaluate the data captured by the first sensor 308, the second sensor 310, or both. For example, the controller 304 and/or the machine controller 306 may receive the images captured by the first sensor 308 and the second sensor 310 for evaluation (e.g., to identify distinct reflectance patterns, determine one or more reflectance values based upon the captured images, and calculate one or more crop-related parameters based upon the determined reflectance value(s)). The machine controller 306 may receive data from, or transmit data to, the controller 304 of the sensor system 302 via the communication device 326 of the machine controller 306. The machine controller 306 may also include a processor 330, a memory 332, and a user interface 334. The processor 330 may be similar to the processor 320 of the controller 304. The memory 332 may be similar to the memory 322 of the controller 304. The user interface 334 may be similar to the user interface 328 of the controller 304 such that an operator may interface with the machine controller 306 to operate the machine, operator the sensor system 302, or both.

The controller 304 and the machine controller 306 may be in communication via a network 336. The network 336 may be or may include, for example, the Internet, a local area network (LAN), a wide area network (WAN), a virtual private network (VPN), or another public or private means of electronic computer communication capable of transferring data between the controller 304 of the sensor system 302 and the machine controller 306. In some implementations, an operator, via the user interface 334 and/or via the user interface 328, may connect to the network 336 via a communal connection point, link, or path, or using a distinct connection point, link, or path. For example, a connection point, link, or path can be wired, wireless, use other communications technologies, or a combination thereof. Additionally, it should be noted that the sensor system 302, the machine controller 306, or other elements of the system 300 may include network hardware such as routers, switches, other network devices, or a combination thereof.

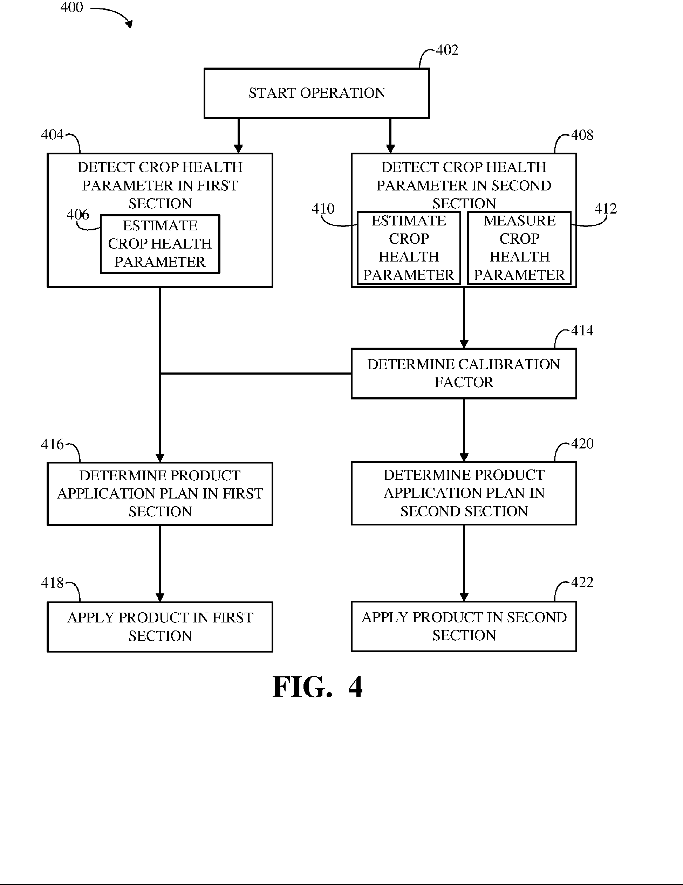

FIG. 4 illustrates an example of a process 400 for agriculture product application using a sensor system. The process 400 may be applicable to the sensor system 106 illustrated in FIGS. 1A and 1B, the sensor system 200 illustrated in FIG. 2, or the system 300 illustrated in FIG. 3. In other words, the process 400 may be implemented, at least in part, by one of the sensor system 106, the sensor system 200, or the system 300. The process 400 can be used to detect and estimate crop health parameters using multiple sensors to determine calibration factors to adjust and formulate tailored product application plans for different crop sections based on the calibration factors, thereby leading to precise and individualized product application to maximize crop health and yield while minimizing excess use of resources.

The process 400 can be performed, for example, by executing a machine-readable program or other computer-executable instructions, such as routines, instructions, programs, or other code. The steps, or operations, of the process 400 may be implemented directly in hardware, firmware, software executed by hardware, circuitry, or a combination thereof. The process 400 can be executed by a processor (e.g., the processor 320 of FIG. 3) associated with or included in the sensor system. The process 400 can also be executed by a processor (e.g., the processor 330 of FIG. 3) of a system in communication with the sensor system (e.g., the machine controller 306 of the system 300).

Initial operation of the sensor system may begin at operation 402 to begin detection of a crop within a field. For illustrative purposes and with reference to FIG. 2, the initial operation of the sensor system 200 may begin at operation 402 to detect one or more parameters (i.e., crop-related parameters) associated with the crop 220. The sensor system 200 may begin detection of one or more parameters associated with the crop 220 in one or more of the sections 222-230 of the crop 220 shown in FIG. 2.

The sensor system may detect a crop health parameter in a first section of the crop (e.g., the first section 222 shown in FIG. 2) at operation 404. The crop health parameter may be any parameter or feature detected by the sensor system that is associated with the crop. For example, the crop health parameter, may be a nutrient level, a moisture level, a fungal pressure, an insect pressure, a crop height, a crop width, other parameters associated with a health of the crop, or a combination thereof. The crop health may be detected at operation 404 using (e.g., based on data captured by) one or more of the sensors. For example, with respect to the sensor system 200, the crop health parameter of the crop 220 in the first section 222 may be detected based upon one or more images captured by the first sensor 204.

Detection of the crop health parameter in the first section at operation 404 may also include estimating the crop health parameter at operation 406. That is, during or after detection of the crop health parameter, the sensor system may capture data (e.g., images) associated with the crop health parameter to estimate a value of the crop health parameter. Estimation of the value of the crop health parameter may be based upon evaluation of the images captured by the sensor system. For example, the images captured by one or more of the sensors within the sensor system may be evaluated to identify distinct reflectance patterns of the crop within the first section, determine one or more reflectance values based upon the captured images, and calculate one or more crop-related parameters based upon the determined reflectance value(s)). By way of example, the first sensor 204 of the sensor system 200 may capture images associated with the crop health parameter of the crop 220 in the first section 222 so that the controller 232 of the sensor system 200 may estimate a value of the crop health parameter of the crop 220 in the first section 222. That is, the value of the crop health parameter based upon the images captured by any of the sensors 204-214, except for the third sensor 208, may be considered the estimated value of the crop health parameter.

The sensor system may detect a crop health parameter in a second section of the crop (e.g., any additional one of the sections 224-230 of the crop 220 shown in FIG. 2) at operation 408. The crop health parameter may be the same crop health parameter detected in the first section at operation 404. That is the crop health parameter detected in the first section at operation 504 may be the same as the crop health parameter detected in the second section at operation 508.

Detection of the crop health parameter in the second section at operation 408 may include estimating the crop health parameter at operation 410 and measuring the crop health parameter at operation 412. That is, during or after detection of the crop health parameter, the sensor system may capture data (e.g., images) associated with the crop health parameter to estimate a value of the crop health parameter and also measure the value of the crop health parameter. Estimation of the value of the crop health parameter may be similar to the estimation completed at operation 406. By way of example, the fourth sensor 210 may capture images associated with the crop health parameter of the crop 220 in the third section 226 so that the controller 232 of the sensor system 200 may estimate a value of the crop health parameter of the crop 220 in the third section 226. That is, the value of the crop health parameter based upon the images captured by the fourth sensor 210 may be considered the estimated value of the crop health parameter.

Additionally, the third sensor 208 may also capture images associated with the crop health parameter of the crop 220 in the third section 226 so that the controller 232 of the sensor system 200 may measure a value of the crop health parameter of the crop 220 in the third section 226. As discussed above, the third sensor 208 and the fourth sensor 210 may be different sensors such that a value of the crop health parameter (e.g., nutrients level) based upon the images captured by the third sensor 208 may be more accurate than a value of the crop health parameter (e.g., nutrients level) based upon the images captured by the fourth sensor 210. That is, the value of the crop health parameter that is determined based upon the images captured by the third sensor 208 may reflect the actual value of the crop health parameter. That is, the value of the crop health parameter based upon the images captured by the third sensor 208 may be considered a measured value of the crop health parameter while the value of the crop health parameter based upon the images captured by any of the other sensors within the sensor system 200 may be considered an estimated value of the crop health parameter.

After estimation and measurement of the crop health parameter at operations 406, 410, and 412, a calibration factor may be determined at 414. The calibration factor may be determined by a controller (e.g., the controller 232 of FIG. 2 or the controller 304 of FIG. 3, the machine controller 234 of FIG. 2 or the machine controller 306 of FIG. 3, or both). The calibration factor may be determined based upon the estimated crop health parameter of the first section of the crop obtained at operation 406, the estimated crop health parameter of the second section of the crop obtained at operation 410, the measured crop health parameter of the second section of the crop obtained at operation 412, or a combination thereof.

By way of example, the estimated crop health parameter obtained at operation 410 may be compared to the measured crop health parameter obtained at operation 412. For example, the estimated crop health parameter obtained at operation 410 may be compared to the measured crop health parameter obtained at operation 412 to determine a difference in value therebetween. The difference may indicate how much greater or smaller the estimated value of the crop health parameter is compared to the measured crop health parameter. As a result, at operation 414, the calibration factor may be determined to define a configuration value. That is, the calibration factor may be a configuration value that at least partially adjusts a product (e.g., fertilizer, fungicide, or pesticide) application plan in a particular section of the crop.

By way of example, the calibration factor determined at operation 414 may establish that the estimated crop health parameter at operation 410 for the second section of the crop is approximately 10% greater in value than the measured crop health parameter obtained at operation 412. In such a case, the calibration factor may be used to adjust a value of the estimated crop health parameter at 410 such that the value of the estimated crop health parameter is equal to the measured crop health parameter at 412. Similarly, since the sensor (e.g., the first sensor 204) used at operation 406 is the same type of sensor used at operation 410 (e.g., the fourth sensor 210), the calibration factor may also adjust a value of the estimated crop health parameter obtained at operation 406 to accommodate for any inaccuracy (e.g., the approximately 10% greater estimated value). The adjusted values may then be utilized to determine product application plans for specific sections of the crop. Alternatively, in lieu of adjusting the value of the estimated crop health parameter based upon the calibration factor to adjust the product application plans, the calibration factor may adjust the product application plans (e.g., operating parameters of the product application plans) directly.

The measured crop health parameter at operation 412, which may be obtained by a more accurate sensor (e.g., the third sensor 208), may be used to determine the calibration factor at operation 414 to adjust measurements of a crop health parameter across all sections being detected (e.g., the sections 222-230). That is, the third sensor 208 may provide a "ground truth" to calibrate data captured by the remaining sensors in the sensor system (e.g., the sensor system 200) to adjust the product application plans. As such, more economical (e.g., cost effective) or different sensors may be used in the sensor system without a loss of accuracy due to the presence of a more accurate sensor (e.g., the third sensor 208). That is, the use of more economical of different sensors in conjunction with the more accurate sensor (e.g., the third sensor 208) may not negatively impact any product application plan that is based upon the estimated crop health parameter. For example, the calibration factor, may be implemented to adjust a product application plan to minimize overapplication and/or underapplication of a particular product.

Once the calibration factor is determined at operation 414, a product application plan as discussed above is determined at 416 for the first section of the crop. The product application plan may be determined by the controller. The product application plan may be or include a process or parameters of application of one or more products to the crop within the first section of the crop. For example, the sensor system 200 may be coupled to an applicator, and the product application plan may include operating conditions of the applicator to apply one or more products to the crop 220 within the first section 222. The one or more products may be fertilizer, nutrients, pesticides, fungicides, water, other topical applications, or a combination thereof. The product application plan may include an application rate, the types of products to be applied, operating conditions of the machine (e.g., speed, direction, ride height, etc.), other operating parameters, or a combination thereof. The product or products may be applied to the first section of the crop (e.g., the first section 222 of the crop 220) at operation 418 in accordance with the application plan determined at operation 416.

Additionally, once the calibration factor is determined at operation 414, a product application plan for the second section of the crop is determined at 420. The product application plan may be determined by the controller. The product application plan may include a process or parameters of application of one or more products to the crop within the second section of the crop, such as described above with respect to operation 416. The product application plan determined at 420 may be different from the product application plan determined at 416. As such, product application for each of the sections of the crop may be tailored and individualized. The product or products may then be applied to the second section of the crop (e.g., the third section 226 of the crop 220) at operation 422 in accordance with the application plan determined at operation 420.

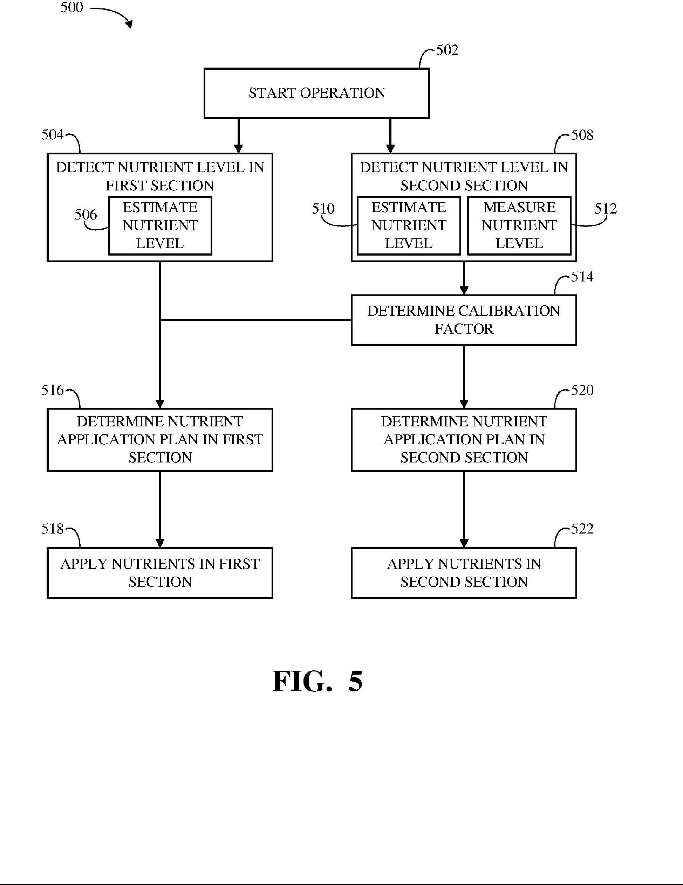

FIG. 5 illustrates another example of a process 500 for agriculture product application using a sensor system. The process 500 may be applicable to the sensor system 106 illustrated in FIGS. 1A and 1B, the sensor system 200 illustrated in FIG. 2, or the system 300 illustrated in FIG. 3. In other words, the process 500 may be implemented, at least in part, by one of the sensor system 106, the sensor system 200, or the system 300. The process 500 can be used to detect and estimate a nutrient level of the crop using multiple sensors to determine calibration factors to adjust and formulate tailored product (e.g., fertilizer or other nutrient application) plans for different crop sections based on the calibration factors, thereby leading to precise and individualized product application to maximize crop health and yield while minimizing excess use of resources.

The process 500 can be performed, for example, by executing a machine-readable program or other computer-executable instructions, such as routines, instructions, programs, or other code. The steps, or operations, of the process 500 may be implemented directly in hardware, firmware, software executed by hardware, circuitry, or a combination thereof. The process 500 can be executed by a processor (e.g., the processor 320 of FIG. 3) associated with or included in the sensor system. The process 500 can also be executed by a processor (e.g., the processor 330 of FIG. 3) of a system in communication with the sensor system (e.g., the machine controller 306 of the system 300).

The process 500 may be similar to the process 400 described above and is intended to illustrate an example of a crop health parameter detected by the sensor system. Initial operation of the sensor system may begin at operation 502. Initiating operation of the sensor system may begin detection of a crop within a field. Initiating operation of the sensor system at operation 502 may be similar to initiating operation of the sensor system at operation 402 of the process 400.

The sensor system may detect a nutrient level in a first section of the crop (e.g., the first section 222 of the crop shown in FIG. 2) at operation 504. The nutrient level may be detected at operation 504 using (e.g., based on data captured by) one or more of the sensors. For example, with respect to the sensor system 200, the nutrient level of the crop 220 in the first section 222 may be detected based upon one or more images captured by the first sensor 204. Detection of the nutrient level at operation 504 may also include estimating the nutrient level at operation 506. Estimating the nutrient level at operation 506 may be similar to the estimation completed at operation 406 or operation 410 of the process 400. For example, the first sensor 204 may capture one or more images of the first section 222 of the crop 220, and the image(s) may be evaluated by the controller 232 to determine the nutrient level. That is, the controller 232 may be configured to identify distinct reflectance patterns of the crop 220 within the image(s) that may be indicative of a particular nutrient level of the crop within the first section 222.

The sensor system may also detect a nutrient level in a second section of the crop (e.g., any additional one of the sections 224-230 of the crop 220 shown in FIG. 2) at operation 508. Detection of the nutrient level in the second section at operation 508 may include estimating the nutrient level at operation 510 and measuring the nutrient level at operation 512. That is, during or after detection of the nutrient level, the sensor system may capture data (e.g., images) associated with the nutrient level to estimate the nutrient level and also measure the nutrient level. Estimation of the nutrient level in the second section may be similar to the estimation completed at operation 410 of the process 400 or at operation 506. Measurement of the nutrient level in the second section may be similar to the measurement completed at operation 412 of the process 400. For example, as discussed above, the measured nutrient level (i.e., a measured crop health parameter) may be a nutrient level that is based upon the image(s) captured by the third sensor 208, whereas the estimated nutrient level (i.e., an estimated crop health parameter) may be a nutrient level that is based upon the image(s) captured by the fourth sensor 210.

After estimation and measurement of the nutrient levels at operation 506, 510, and 512, a calibration factor may be determined at 514. The calibration factor may be determined by a controller (e.g., the controller 232 of FIG. 2 or the controller 304 of FIG. 3, the machine controller 234 of FIG. 2 or the machine controller 306 of FIG. 3, or both). The calibration factor may be determined in a similar manner to the calibration factor determined at 414 in the process 400 described above. For example, the calibration may be determined by comparing the measured nutrient level obtained at operation 512 to the estimated nutrient level obtained at operation 510.

Once the calibration factor is determined at operation 514, a nutrient application plan for the first section of the crop is determined at 516. The nutrient application plan may be determined by the controller. The nutrient application plan may be a method or parameters of nutrient application within the first section of the crop, which may be similar to the product application plan determined at operation 416 of the process 400. The nutrients may be applied to the first section of the crop at operation 518 in accordance with the application plan determined at operation 516.

Additionally, once the calibration factor is determined at operation 514, a nutrient application plan for the second section of the crop is determined at 520. The nutrient application plan may be determined by the controller. The nutrient application plan may be a method or parameters of nutrient application to the crop within the second section of the crop, which may be similar to the product application plan determined at operation 420 of the process 400. The nutrients may then be applied to the second section of the crop (at operation 522 in accordance with the application plan determined at operation 520.

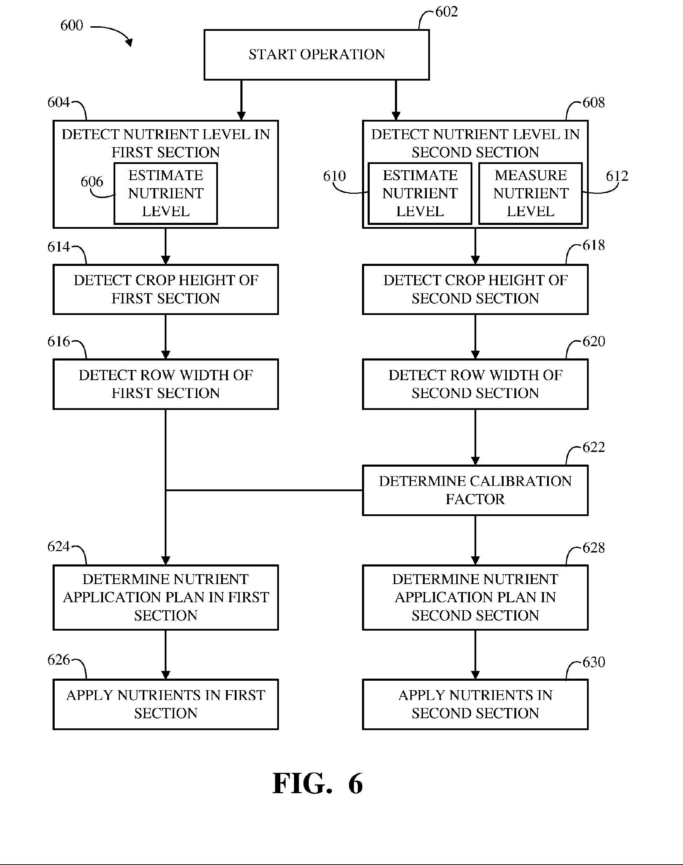

FIG. 6 illustrates another example of a process 600 agriculture product application using a sensor system. The process 600 may be applicable to the sensor system 106 illustrated in FIGS. 1A and 1B, the sensor system 200 illustrated in FIG. 2, or the system 300 illustrated in FIG. 3. In other words, the process 600 may be implemented, at least in part, by one of the sensor system 106, the sensor system 200, or the system 300. Similar to the process 500 described above, the process 600 can be used to detect and estimate a nutrient level of the crop using multiple sensors to determine calibration factors to adjust and formulate tailored nutrient application plans. In this particular case, the process 600 may also use crop height and/or crop row width to formulate the nutrient application plans.

The process 600 can be performed, for example, by executing a machine-readable program or other computer-executable instructions, such as routines, instructions, programs, or other code. The steps, or operations, of the process 500 may be implemented directly in hardware, firmware, software executed by hardware, circuitry, or a combination thereof. The process 600 can be executed by a processor (e.g., the processor 320 shown in FIG. 3) associated with or included in the sensor system. The process 600 can also be executed by a processor (e.g., the processor 330 of FIG. 3) of a system in communication with the sensor system (e.g., the machine controller 306 of the system 300).

The process 600 may be similar to the process 400 and the process 500 described above and is intended to illustrate another example of a crop health parameter detected by the sensor system. Initiating operation of the sensor system at operation 602 may be similar to initiating operation of the sensor system at operation 502.

The sensor system may detect a nutrient level in a first section of the crop (e.g., the first section 222 of the crop shown in FIG. 2) at operation 604. The nutrient level may be detected at operation 604 using (e.g., based on data captured by) one or more of the sensors. Detection of the nutrient level at operation 604 may also include estimating the crop health parameter at operation 606. Estimating the nutrient level at operation 606 may be similar to the estimation completed at operation 506 of the process 400.

The sensor system may also detect a nutrient level in a second section of the crop (e.g., any additional one of the sections 224-230 of the crop 220 shown in FIG. 2) at operation 608. Detection of the nutrient level in the second section at operation 608 may include estimating the nutrient level at operation 610 and measuring the nutrient level at operation 612. That is, during or after detection of the nutrient level, the sensor system may capture data (e.g., images) associated with the nutrient level to estimate the nutrient level and also measure the nutrient level. Estimation of the nutrient level in the second section may be similar to the estimation completed at operation 510 of the process 500. Measurement of the nutrient level in the second section may be similar to the measurement completed at operation 512 of the process 500. For example, as discussed above, the measured nutrient level (i.e., a measured crop health parameter) may be a nutrient level that is based upon the image(s) captured by the third sensor 208, whereas the estimated nutrient level (i.e., an estimated crop health parameter) may be a nutrient level that is based upon the image(s) captured by the fourth sensor 210.

The process 600 may also include detecting a crop height of the crop within the first section at operation 614 and detecting a row width of the first section of the crop at operation 616. The crop height and the row width may be detected by one or more sensors of the sensor system and/or by one or more additional sensors, such as an additional height or width detection sensor that is part of the sensor system or in communication with the sensor system.

By way of example, one or more sensors may capture multiple, overlapping images of the crop within the first section from different angles. The images may then be evaluated by the processor of the sensor system or the machine processor in communication with the sensor system to identify common points between the overlapping images, and the common points may then be utilized to reconstruct a three-dimensional model of the first section of the crop. The three-dimensional model may then be measured to determine the crop height and/or the row width. Additionally, or alternatively, one or more sensors may implement light detection and ranging (LiDAR) to emit laser pulses towards the first section of the crop. The one or more sensors may detect the reflected laser pulses from the ground and the crop to determine distances, and the determined distances may then be utilized to reconstruct a three-dimensional model of the first section of the crop. The three-dimensional model may then be measured to determine the crop height and/or the row width.

The process 600 may further include detecting a crop height of the crop within the second section at operation 618 and detecting a row width of the second section of the crop at operation 620. The crop height and the row width may be detected by one or more sensors of the sensor system and/or by one or more additional sensors, such as an additional height or width detection sensor that is part of the sensor system or in communication with the sensor system. Determining the crop height at operation 618 may be similar to determining the crop height at operation 614. Determining the row width at operation 620 may be similar to determining the row width at operation 616.

After detection of the crop height and row width of both the first section and the second section, a calibration factor may be determined at 622. The calibration factor may be determined by a controller of the sensor system by a machine controller of a machine in communication with the sensor system, or both. The calibration factor may be determined in a similar manner to the calibration factor determined at 414 in the process 400 and/or the calibration factor determined at 514 in the process 500 described above. Additionally, the calibration factor may also utilize the crop height and/or row width determined for both the first section and the second section of the crop based upon operations 614-620. That is, the calibration factor may be determined at least partially based upon the nutrient level, crop height, and row width determined in the first section and the second section.

Once the calibration factor is determined at operation 622, a nutrient application plan for the first section of the crop is determined at 624. The nutrient application plan may be determined by the controller. The nutrient application plan may be a method or parameters of nutrient application within the first section of the crop, which may be similar to the nutrient application plan determined at operation 516 of the process 500. The nutrients may be applied to the first section of the crop at operation 626 in accordance with the application plan determined at operation 624.

Additionally, once the calibration factor is determined at operation 622, a nutrient application plan for the second section of the crop is determined at 628. The nutrient application plan may be determined by the controller. The nutrient application plan may be a method or parameters of nutrient application to the crop within the second section of the crop, which may be similar to the product application plan determined at operation 520. The nutrients may then be applied to the second section of the crop at operation 630 in accordance with the application plan determined at operation 628.

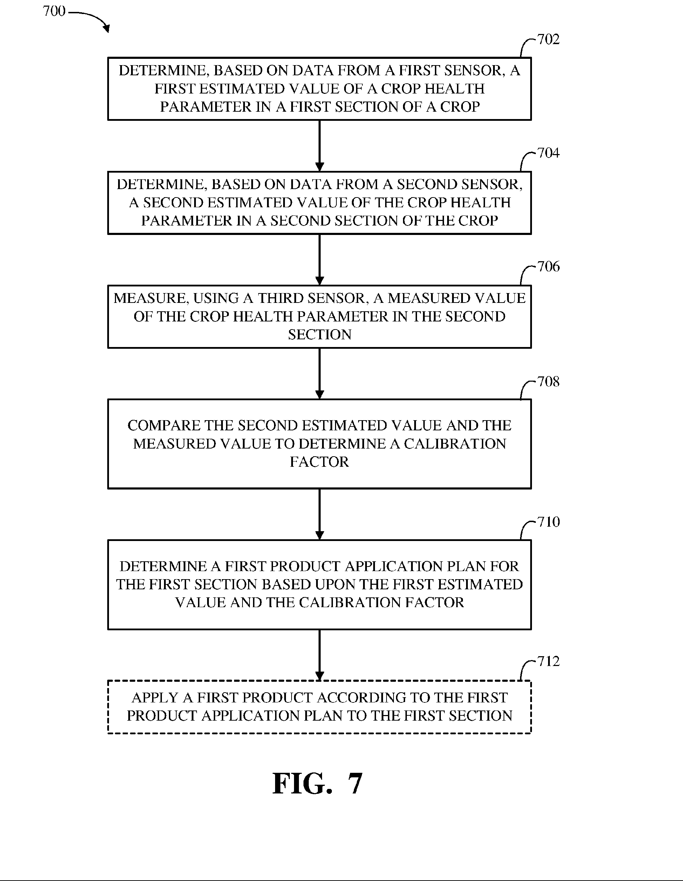

FIG. 7 illustrates an example of a process 700 of agriculture product application using a sensor system. The process 700 may be performed with respect to and/or by the sensor system 106 illustrated in FIGS. 1A and 1B, the sensor system 200 illustrated in FIG. 2, or the system 300 illustrated in FIG. 3. In other words, the process 700 may be at least partially completed by the sensor system 106, the sensor system 200, or the system 300.

The process 700 can be performed, for example, by executing a machine-readable program or other computer-executable instructions, such as routines, instructions, programs, or other code. The steps, or operations, of the process 700 may be implemented directly in hardware, firmware, software executed by hardware, circuitry, or a combination thereof. The process 700 can be executed by a processor (e.g., the processor 320 of the sensor system 302 shown in FIG. 3) associated with or included in the sensor system. The process 700 can also be executed by a processor (e.g., the processor 330) of a system in communication with the sensor system (e.g., the machine controller 306 of the system 300). The process 700 may be similar to the processes 400, 500, and 600 described above.

The process 700 includes determining a first estimated value of a crop health parameter in a first section of a crop at step 702. The crop health parameter may be any parameter associated with the crop, such as a nutrient level, moisture level, fungal presence, insect presence, or other crop health parameter. The first estimated value of the crop health parameter may be determined based on data (e.g., images) obtained from a first sensor. For example, the first sensor 204 of the sensor system 200 may obtain data, which may in turn be evaluated by the controller 232 to estimate the value of the crop health parameter (e.g., nutrient level) in the first section 222 of the crop 220.

The process 700 may also include determining a second estimated value of the crop health parameter in a second section of the crop at step 704. The second estimated value of the crop health parameter may be determined based on data (e.g., images) obtained from a second sensor. For example, the fourth sensor 210 of the sensor system 200 may obtain data, which may in turn be evaluated by the controller 232 to estimate the value of the crop health parameter (e.g., nutrient level) in the third section 226 of the crop 220.

The method may also include measuring a measured value of the crop health parameter in the second section of the crop at step 706. The measured value of the crop health parameter may be determined based on data (e.g., images) obtained from a third sensor. For example, the third sensor 208 of the sensor system 200, which may be a different type of sensor than the first sensor and the second sensor, may obtain data, which may in turn be evaluated by the controller 232 to measure the crop health parameter (e.g., nutrient level) in the third section 226 of the crop 220.

The method may further include comparing the second estimated value and the measured value to determine a calibration factor at step 708. The calibration factor may be determined similar to the calibration factor of the process 400, the calibration factor of the process 500, and the calibration factor of the process 600 described above.

After determining the calibration factor at step 708, a first product application plan for the first section may be determined at step 710. The first product application plan may be based upon the first estimated value and the calibration factor. The first product application plan may be similar to the product application plan of the process 400 described above, whereby a product or products (e.g., nutrients) may be applied to the first section of the crop based upon the first product application plan at step 712. However, in certain implementations, the process 700 may not include the step 712.

It should also be noted that while FIGS. 4-7 discussed above reference a first section and a second section of the crop, the processes and methods above may also be implemented in any number of sections of the crop. For example, with respect to FIG. 2, the processes and methods discussed with respect to FIGS. 4-7 may be implemented in all or a portion of the sections 222-230 of the crop 220. The sensors 204-214 may form an array of sensors that extend across a working width of the apparatus 202. That is, a combined width of each of the sections 222-230 may form the working width of the apparatus 202, and the array of sensors may be used to determine various crop health parameters along a portion or an entirety of the working width.

While the disclosure has been described in connection with certain embodiments of a sensor system, it is to be understood that the disclosure is not limited to or by the disclosed embodiments but, on the contrary, is intended to cover various modifications and equivalent arrangements included within the scope of the appended claims, which scope is to be accorded the broadest interpretation so as to encompass all such modifications and equivalent structures as is permitted under the law.

Persons skilled in the art will understand that the various embodiments of the present disclosure and shown in the accompanying figures constitute non-limiting examples, and that additional components and features may be added to any of the embodiments discussed hereinabove without departing from the scope of the present disclosure. Additionally, persons skilled in the art will understand that the elements and features shown or described in connection with one embodiment may be combined with those of another embodiment without departing from the scope of the present disclosure to achieve any desired result and will appreciate further features and advantages of the presently disclosed subject matter based on the description provided. Variations, combinations, and/or modifications to any of the embodiments and/or features of the embodiments described herein that are within the abilities of a person having ordinary skill in the art are also within the scope of the present disclosure, as are alternative embodiments that may result from combining, integrating, and/or omitting features from any of the disclosed embodiments.

Use of the term "optionally" with respect to any element of a claim means that the element may be included or omitted, with both alternatives being within the scope of the claim. Additionally, use of broader terms such as "comprises," "includes," and "having" should be understood to provide support for narrower terms such as "consisting of," "consisting essentially of," and "comprised substantially of." Accordingly, the scope of protection is not limited by the description set out above, but is defined by the claims that follow, and includes all equivalents of the subject matter of the claims.

In the preceding description, reference may be made to the spatial relationship between the various structures illustrated in the accompanying drawings, and to the spatial orientation of the structures. However, as will be recognized by those skilled in the art after a complete reading of this disclosure, the structures described herein may be positioned and oriented in any manner suitable for their intended purpose. Thus, the use of terms such as "above," "below," "upper," "lower," "inner," "outer," "left," "right," "upward," "downward," "inward," "outward," "horizontal," "vertical," etc., should be understood to describe a relative relationship between the structures and/or a spatial orientation of the structures. Those skilled in the art will also recognize that the use of such terms may be provided in the context of the illustrations provided by the corresponding figure(s).

Additionally, terms such as "approximately," "generally," "substantially," and the like should be understood to allow for variations in any numerical range or concept with which they are associated and encompass variations on the order of 25% (e.g., to allow for manufacturing tolerances and/or deviations in design). For example, the term "generally parallel" should be understood as referring to configurations in with the pertinent components are oriented so as to define an angle therebetween that is equal to 180° ± 25% (e.g., an angle that lies within the range of (approximately) 135° to (approximately) 225°). The term "generally parallel" should thus be understood as referring to encompass configurations in which the pertinent components are arranged in parallel relation.

Although terms such as "first," "second," "third," etc., may be used herein to describe various operations, elements, components, regions, and/or sections, these operations, elements, components, regions, and/or sections should not be limited by the use of these terms in that these terms are used to distinguish one operation, element, component, region, or section from another. Thus, unless expressly stated otherwise, a first operation, element, component, region, or section could be termed a second operation, element, component, region, or section without departing from the scope of the present disclosure.

Each and every claim is incorporated as further disclosure into the specification and represents embodiments of the present disclosure. Also, the phrases "at least one of A, B, and C" and "A and/or B and/or C" should each be interpreted to include only A, only B, only C, or any combination of A, B, and C.

## Claims

1. A method, comprising:
determining, based on data from a first sensor (204), a first estimated value of a crop health parameter in a first section (222) of a crop (220);
determining, based on data from a second sensor (206), a second estimated value of the crop health parameter in a second section (224) of the crop (220);
measuring, using a third sensor (208), a measured value of the crop health parameter in the second section (224);
comparing the second estimated value and the measured value to determine a calibration factor; and
determining a first product application plan for the first section (222) based upon the first estimated value and the calibration factor.

2. The method of claim 1, further comprising:
applying a first product according to the first product application plan to the first section (222).

3. The method of claim 1 or claim 2, wherein the first estimated value is a first estimated nutrient level in the first section (222), the second estimated value is a second estimated nutrient level in the second section (224), and the measured value is a measured nutrient level in the second section (224).

4. The method of any one of the preceding claims, wherein the first sensor (204) and the second sensor (206) are visible light cameras and the third sensor (208) is a multispectral camera.

5. The method of any one of claims 1 to 3, wherein the first sensor (204) and the second sensor (206) are visible light cameras and the third sensor (208) is a near-infrared camera.

6. The method of any one of claims 1 to 3, wherein the first sensor (204) and the second sensor (206) are multispectral cameras and the third sensor (208) is a near-infrared camera that includes a calibrated light source.

7. The method of any one of claims 1 to 3, wherein the first sensor (204) and the second sensor (206) are near-infrared cameras and the third sensor (208) is a near infrared sensor that includes a calibrated light source.

8. The method of any one of the preceding claims, further comprising:
determining a second product application plan for the second section (224) based upon the second estimated value and the calibration factor; and
applying a second product according to the second product application plan to the second section (224).

9. The method of any one of the preceding claims, wherein the first sensor (204) and the second sensor (206) form an array of sensors that extends across a working width of a machine (100), and a width of the first section (222) combined with a width of the second section (224) form the working width of the machine (100).

10. The method of any one of the preceding claims, further comprising:
detecting, using a height sensor, a height of the crop (220) within the first section (222),
wherein the first product application plan is further based upon the height of the crop (220).

11. The method of claim 10, further comprising:
detecting, using the first sensor (204), a row width of the crop (220) within the first section (222),
wherein the first product application plan is further based upon the row width of the crop (220).

12. The method of any one of claims 1 to 9, further comprising:
detecting, using a height sensor, a height of the crop (220) within the first section (222); and
detecting, using the first sensor (204), a row width of the crop (220) within the first section (222),
wherein determining the first product application plan is further based upon the height of the crop (220) and the row width of the crop (220).

13. The method of any one of the preceding claims, wherein the calibration factor corresponds to a difference between the second estimated value and the measured value.

14. The method of any one of the preceding claims, further comprising:
determining a second product application plan for the second section (224) based upon the second estimated value and the calibration factor or based upon the measured value; and
applying a second product according to the second product application plan to the second section (224).

15. The method of any one of the preceding claims, further comprising:
transmitting, via a network (336), information associated with the crop (220) to a controller (306) of a vehicle (100), wherein the information associated with the crop (220) includes at least one of the first estimated value, the second estimated value, the measured value, the calibration factor, and the first product application plan.
